Europäisches Patentamt

European Patent Office

Office européen des brevets

(11) Veröffentlichungsnummer: **0 331 932**
**A2**

(12) **EUROPÄISCHE PATENTANMELDUNG**

(21) Anmeldenummer: 89102480.4

(51) Int. Cl.4: **A61M 25/00**

(22) Anmeldetag: 14.02.89

(30) Priorität: 09.03.88 DE 8803153 U

(43) Veröffentlichungstag der Anmeldung:
13.09.89 Patentblatt 89/37

(84) Benannte Vertragsstaaten:
AT DE ES FR GB IT LU NL

(71) Anmelder: **B. Braun Melsungen AG**
**Carl-Braun Strasse**
**D-3508 Melsungen(DE)**

(72) Erfinder: **Witt, Hans-Hinrich, Dr.**
**Buchenhain 15**
**D-3501 Körle(DE)**

(74) Vertreter: **Selting, Günther, Dipl.-Ing. et al**
**Patentanwälte von Kreisler, Selting, Werner**
**Deichmannhaus am Hauptbahnhof**
**D-5000 Köln 1(DE)**

(54) Katheterset für die Plexusanästhesie.

(57) Die Erfindung bezweckt die Schaffung eines Kathetersets für die Plexusanästhesie, bei dem die Verlegung des Katheters unter elektrischer Stimulation möglich ist und der auch nachträglich eine Kontrolle der Katheterlage gestattet. Der Katheterset weist eine Punktionskanüle (10), ein auf die Punktionskanüle (10) aufgeschobenes Kapillar (14) und einen durch das Kapillar (14) einführbaren Katheter mit offenem patientenseitigen Ende auf. In den Katheter kann ein Führungsdraht eingeschoben werden, der am patientenfernen Ende eine elektrische Kontakteinrichtung aufweist und dessen patientenseitiges Ende blank ist. Durch elektrische Impulse kann die Lage des Führungsdrahtes bzw. des Katheterendes in der Fascie überwacht werden. Wenn der Katheter über längere Zeit verlegt bleibt, ist es möglich, durch nachträgliches Einführen des Führungsdrahtes jederzeit die Katheterlage zu kontrollieren und erforderlichenfalls zu korrigieren.

FIG.1

## Katheterset für die Plexusanästhesie

Die Erfindung betrifft einen Katheterset für die Plexusanästhesie nach dem Oberbegriff des Anspruchs 1.

Ein Katheterset von dem der Oberbegriff des Anspruchs 1 ausgeht, ist aus DE-GM 82 22 222 bekannt. Bei der bekannten Vorrichtung ist auf eine Punktionskanüle, die am vorderen Ende mit einem geeigneten Schliff versehen ist, ein Kunststoff-Kapillar aufgeschoben. Nach dem Punktieren des Nervengefäßes wird die Punktionskanüle aus dem Kapillar herausgezogen und in das Kapillar wird ein Katheter eingeführt, durch den ein Anästhetikum dem Patienten zugeführt werden kann. Nachteilig ist bei dem bekannten Plexuskatheter, daß das Aufsuchen des zu behandelnden Nervs mit dem Katheter sehr schwierig ist und viel Erfahrung und Geschick erfordert. Außerdem kann das Kapillar und/oder der Katheter abknicken, wodurch das Vorschieben des Katheters behindert wird.

Bekannt ist auch ein Katheterset für die Plexusanästhesie, bei welchem ein mit Kunststoff überzogener Mandrin benutzt wird, um die Steifigkeit des Katheters bei der Verlegung zu erhöhen. Hierbei können gerade wegen der Steifigkeit des Mandrins feine Nervenabzweigungen verletzt werden. Eine exakte Kontrolle der Katheterlage ist auch bei diesem Katheterset nicht möglich.

Der Erfindung liegt die Aufgabe zugrunde, einen Katheterset der im Oberbegriff des Anspruchs 1 angegebenen Art zu schaffen, bei dem die Verlegung des Katheters unter elektrischer Stimulation möglich ist und der auch nachträglich eine Kontrolle der Katheterlage gestattet.

Die Lösung dieser Aufgabe erfolgt mit den im kennzeichnenden Teil des Anspruchs 1 angegebenen Merkmalen.

Bei dem erfindungsgemäßen Katheterset ist ein in den Katheter einschiebbarer Führungsdraht vorhanden, der einerseits eine versteifende Wirkung auf den Katheter ausübt und dessen Verlegung erleichtert, andererseits aber auch als elektrischer Leiter für die Neurostimulation wirkt. Das vordere Ende des Führungsdrahts, das geringfügig aus dem Katheter herausragt ist blank und am rückwärtigen Ende befindet sich eine Kontakteinrichtung. Auf diese Weise kann durch elektrische Impulse die Lage des Führungsdrahts bzw. des Katheterendes in der Fascie überwacht werden. Wenn der Katheter über längere Zeit verlegt bleibt, ist es möglich, durch nachträgliches Einführen des Führungsdrahtes jederzeit die Katheterlage zu kontrollieren und erforderlichenfalls zu korrigieren.

Die gemäß Anspruch 2 vorgesehene Markierung an dem Führungsdraht gibt an, wann nur noch die Drahtspitze aus dem isolierenden Katheter herausteht. Auf diese Weise können die patientenseitigen Enden von Plexuskatheter und Führungsdraht aufeinander abgestimmt werden. Das blanke vordere Ende des Führungsdrahts ist von dem Katheter nicht umschlossen, so daß es mit dem Gewebe in Berührung kommt. Durch Nervenstimulation mit Hilfe des Führungsdrahts kann nach Tagen oder Wochen immer wieder die korrekte Katheterlage überprüft werden.

In den Unteransprüchen 3 bis 5 sind zweckmäßige Weiterbildungen der Erfindung angegeben.

Im folgenden wird unter Bezugnahme auf die Zeichnungen ein Ausführungsbeispiel der Erfindung näher erläutert. Es zeigen:

Fig. 1 eine Darstellung der Punktionskanüle mit aufgeschobenem Kapillar,

Fig. 2 den Katheter mit zugehörigem Führungsdraht und

Fig. 3 in vergrößertem Maßstab eine Darstellung der beiden Endabschnitte des Führungsdrahtes.

Der Katheterset weist eine aus Stahl bestehende Punktionskanüle 10 auf, die am vorderen Ende mit einem speziellen Schliff 11 versehen ist, welcher das Durchstoßen der Fascienwand ohne Zerstörung der Nervenfasern ermöglicht. Am rückwärtigen Ende der Kanüle 10 befindet sich ein Kanülenansatz 12 mit Griffstück 13. Auf die Kanüle 10 ist das aus Kunststoff bestehende Kapillar 14 aufgeschoben, das als Einführvorrichtung für den Katheter benutzt wird. Das vordere Ende des Kapillars 14 befindet sich in geringem Abstand hinter dem Schliff 11 und am rückwärtigen Ende befindet sich ein aus Kunststoff bestehendes Ansatzstück 15, das mit dem Kanülenansatz 12 zusammengreift und durch eine Luer-Lock-Verbindung verriegelbar ist. Am rückwärtigen Ende des Kanülenansatzes 12 ist ein durch einen Stopfen 16 verschlossenes Anschlußstück 17 vorgesehen, um während der Punktion der Nerven-Gefäß-Scheidewand eine Spritze an die Kanüle 10 anschließen zu können.

Die metallische Kanüle 10 ist elektrisch über ein Kabel 18 mit einem Stecker 19 verbunden, der an einen (nicht dargestellten) Nervenstimulator angeschlossen werden kann.

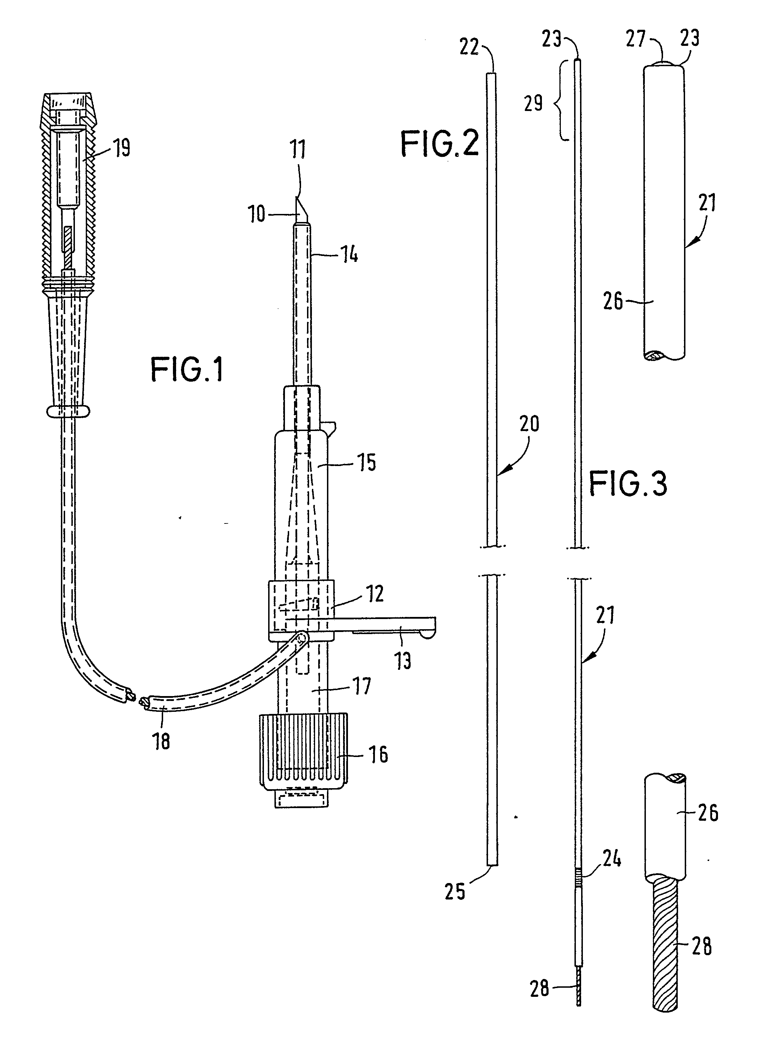

In dem in Fig. 1 dargestellten Zustand wird mit der Punktionskanüle 10 der Nerv in der Fascienhülle unter elektrischer Stimulation aufgesucht. Dabei werden der Punktionskanüle 10 elektrische Impulse zugeführt. Befindet sich das aus dem Kapillar 14 herausragende Ende der Punktionskanüle in unmittelbarer Nähe des betreffenden Nervs, z.B. in einem Abstand von weniger als 1 bis 2 mm, dann wird auf den Nerv eine Reizung ausgeübt, die zu einer Reflexbewegung des betreffenden Gliedes, z.B. eines Fingers, führt.

Nach dem Entfernen der Punktionskanüle 10 verbleibt das Kapillar 14 als Einführhilfe für den Katheter 20 im Körper des Patienten. Der Katheter 20 wird auf den Führungsdraht 21 aufgeschoben und in diesem Zustand durch das Kapillar 14 hindurch in die Fascie eingeführt. Dabei liegen die vorderen Enden 22,23 von Katheter 20 und Führungsdraht 21 etwa bündig, wobei das Führungsdrahtende 23 geringfügig aus dem Katheterende 22 herausragt. Dieser Zustand wird dadurch festgestellt, daß im rückwärtigen Bereich des Führungsdrahts 21 eine Farbmarkierung 24 angebracht ist, die das rückwärtige Ende 25 des Katheters 20 erreicht, wenn die vorderen Enden 22 und 23 sich in der richtigen Zuordnung zueinander befinden. Der Führungsdraht 21 ist bei dem vorliegenden Ausführungsbeispiel mit einer elektrischen Isolierschicht 26 versehen, die sich über die gesamte Länge erstreckt und lediglich das vordere blanke Drahtende 27 und einen am rückwärtigen Ende vorgesehenen abisolierten Abschnitt 28 freiläßt, welcher die Kontakteinrichtung bildet. Über die Katheterlänge verteilt sind verschiedene Markierungen am Katheter angebracht, um die Eindringtiefe des Katheters in bezug auf das Ansatzstück 15 ermitteln zu können.

Der Führungsdraht 21 erlaubt eine exakte Lagebestimmung des vorderen Katheterendes 22 durch Neurostimulation, wobei der Neurostimulator mit der Kontakteinrichtung 28 verbunden wird und elektrische Impulse, die von dem blanken Ende 27 ausgehen, auf das umgebende Gewebe einwirken. Auf diese Weise erfolgt die Neurostimulation über den Führungsdraht 21 und damit eine Ortsbestimmung der Katheteröffnung 22 zum Nervenplexus. Der Führungsdraht 21 wirkt gleichzeitig als Versteifungselement für den Katheter 20 und sein vorderer Endabschnitt 29 ist weicher als die übrige Führungsdrahtlänge, um Beschädigungen empfindlicher feiner Nervenabzweigungen zu vermeiden.

Nachdem der Katheter plaziert wurde, kann der Führungsdraht 21 aus dem Katheter 20 herausgezogen werden.

Eine Lagekontrolle des Katheters 20 ist jederzeit mit einem neuen Führungsdraht 21, der in den Katheter eingeschoben wird, möglich. Auf diese Weise kann mittels Neurostimulation über den Führungsdraht die Katheterlage kontrolliert und ggf. durch Verschieben von Katheter und Führungsdraht verändert werden.

Der Katheter 20 hat die bei Plexuskathetern übliche geringe Dicke mit einem Außendurchmesser von etwa 1,05 mm und einem Innenlumen von 0,65 mm. Der Durchmesser des zugehörigen Führungsdrahts 21 beträgt 0,6 mm, so daß der Führungsdraht das Katheterlumen ausfüllt, jedoch ohne wesentliche Reibung im Katheter verschoben werden kann.

## Ansprüche

1. Katheterset für die Plexusanästhesie, mit
- einer Punktionskanüle (10),
- einem auf die Punktionskanüle (10) aufschiebbaren oder darauf festsitzenden Kunststoff-Kapillar (14),
- und einem durch das Kapillar (14) einführbaren Katheter (20) mit offenem patientenseitigen Ende (22),
dadurch gekennzeichnet, daß ein in den Katheter (20) einschiebbarer Führungsdraht (21) vorgesehen ist, der am patientenfernen Ende eine elektrische Kontakteinrichtung (28) aufweist und dessen patientenseitiges Ende (27) blank ist.

2. Katheterset nach Anspruch 1, dadurch gekennzeichnet, daß der Führungsdraht (21) an einer etwa der Katheterlänge entsprechenden Stelle eine Markierung (24) zur Begrenzung der Einführtiefe auf ein Maß, bei dem das Ende (23) des Führungsdrahts (21) das vordere Katheterende (22) erreicht hat.

3. Katheterset nach Anspruch 1 oder 2, dadurch gekennzeichnet, daß der vordere Endbereich (29) des Führungsdrahts (21) weicher ist als die übrige Länge des Führungsdrahts.

4. Katheterset nach einem der Ansprüche 1 bis 3, dadurch gekennzeichnet, daß der Führungsdraht (21) eine isolierende Umhüllung (26) aufweist, die nur das vordere und das rückwärtige Ende (27,28) des Drahtes freiläßt.

5. Katheterset nach einem der Ansprüche 1 bis 4, dadurch gekennzeichnet, daß die Punktionskanüle (10) mit einem Anschlußelement (19) zum elektrischen Anschluß eines Neurostimulators verbunden ist.

EP 0 331 932 A2

FIG.1

FIG.2

FIG.3

10  11  14  15  12  13  17  16  18  19

22  23  27  23  29  21  26  20  25  21  24  26  28  28